# EUROPEAN PATENT APPLICATION

(11) **EP 2 210 903 A1**
(43) Date of publication of application: **28.07.2010**
(21) Application number: 09305057.3
(22) Date of filing: 21.01.2009
(51) Int. Cl.: C07K 16/28, A61K 39/395, A61P 9/10, A61P 37/06, C07K 16/46

(54) **Anti-CD160 monoclonal antibodies and uses thereof**

(71) Applicant: MONOCLONAL ANTIBODIES THERAPEUTICS, 91000 Evry (FR)
(72) Inventor: Didierlaurent, Denis, 93390 Clichy-sous-Bois (FR); Chose, Olivier, 75020 Paris (FR); Kadouche, Jean, 75013 Paris (FR)
(74) Representative: Ahner, Francis

(57) **Abstract**

The present application concerns an antibody or fragment thereof; capable of binding to CD 160, said antibody comprising a) a light chain comprising three light chain complementary regions (CDRs) having the following amino acid sequences: (i) the light chain CDR1: QSISNH (SEQ ID NO: 1), (ii) the light chain CDR2: YAS, (iii) the light chain CDR3: QQSNSWPLT (SEQ ID NO: 2), and a light chain framework sequence from an inimunoglobulin light chain; and b) a heavy chain comprising three heavy chain complementary regions (CDRs) having the following amino acid sequences: (i) the heavy chain CDR1: GYTFTDYW (SEQ TD NO: 3), (ii) the heavy chain CDR2: IYPGDDDA (SEQ ID NO: 4); (iii) the heavy chain CDR3: ARRGIAAVVGGFDY (SEQ ID NO: 5); and a heavy chain framework sequence from an immunoglobulin heavy chain; a pharmaceutical comprising said antibody and the use of said antibody for preparation of a medieament for treating and/or preventing a pathology associated with endothelial cells proliferation engaged in an angiogenesis process.

## Description

### FIELD OF THE INVENTION:

The present invention provides novel anti-CD160 monoclonal antibodies, in particular mouse-human chimeric anti-CD160 antibodies. Anti-CD160 antibodies, as well as pharmaceutical compositions containing them, are useful for inhibiting angiogenesis.

### BACKGROUND OF THE INVENTION:

NK cells major effector functions are characterized by cytolytic activity and the production of cytokines and chemokines directed against susceptible target cells. NK cell discriminate between healthy and abnormal tumor cells or virally infected cells through specific engagement of various activating receptors. Most activating NK cell receptors can be divided in three groups according their signalling mediated associated molecules, but a unique distinct activating NK cell receptor has been identified, CD160, which is an MHC class I-dependent immunoglobulin (Ig)-like molecule.

CD160 is expressed by human and mouse circulating cytotoxic lymphocytes, as NK CD56^{dim+}CD16⁺, most TCRγδ T cells, and cytotoxic effector TCR αβ CD8T cells, and also in almost all intestinal intraepithelial lymphocytes (iIELs) (MAIZA et al., J. Exp. Med., vol. 178, p: 1124-6, 1993; ANUMANTHAN et al., J. Immunol., vol.161, p:2780-90, 1998).

Finally, CD160 exhibits the following unique characteristics when compared to the other activating receptors described to date: 1) CD 160 is encoded by a gene on chromosome 1 outside the NK gene complex, 2) it is a multimeric glycosyl phosphatidyl inositol (GPI)-anchored molecule, 3) its cell surface expression is down-modulated after activation.

Angiogenesis is a fundamental process by means of which new capillaries from the pre-existing blood vessels are formed. This process is essential in many normal physiological phenomena such as reproduction, differentiation, cicatrisation, and organ regeneration.

Angiogenesis is under strict control in these normal biological phenomena; i.e., it is triggered during a brief period of several days and then completely inhibited. However, many pathologies are linked to invasive, uncontrolled angiogenesis. Arthritis, for example, is a pathology caused by damage caused to cartilage by invasive neovessels. In diabetic retinopathy, the invasion of the retina by neovessels results in the patients going blind; neovascularization of the ocular apparatus is the major cause of blindness and this neovascularization dominates at least twenty diseases of the eye. Lastly, the growth and metastasis of many tumors are directly dependent on angiogenesis. The tumor stimulates the growth of the neovessels for its own use. Furthermore, these neovessels present escape routes by means of which the tumors can reach the blood circulatory system and cause metastases in remote sites such as the liver, lungs or bones.

Recently, CD 160 has been identified as an inhibitory signalling receptor for angiogenesis (FONS et al., Blood, vol.108(8), p: 2608-15, 2006).

WO 03/018048 in the name of ABTECH and INSERM relates to the use of two soluble HLA class I molecules that bind CD160, namely sHLA-G1 and sHLA-B7, to inhibit angiogenesis.

WO 2006/015886 in the name of INSERM relates to the use of an anti-CD160 monoclonal antibody for inhibiting vessel formation and growth that is induced by pro-angiogenic factors such as VEGF or FGF2 on endothelial cells.

### SUMMARY OF THE INVENTION:

The present invention relates to an antibody, or fragment thereof, capable of binding to CD160, said antibody comprising:
a) a light chain comprising three light chain complementary regions (CDRs) having the following amino acid sequences:
   i) the light chain CDR1: QSISNH (SEQ ID NO:1);
   ii) the light chain CDR2: YAS;
   iii) the light chain CDR3: QQSNSWPLT (SEQ ID NO: 2); and
   a light chain framework sequence from an immunoglobulin light chain; and
b) a heavy chain comprising three heavy chain complementary regions (CDRs) having the following amino acid sequences:
   i) the heavy chain CDR1: GYTFTDYW (SEQ ID NO: 3);
   ii) the heavy chain CDR2: IYPGDDDA (SEQ ID NO: 4);
   iii) the heavy chain CDR3: ARRGIAAVVGGFDY (SEQ ID NO: 5); and
   a heavy chain framework sequence from an immunoglobulin heavy chain.

The present invention also relates to a pharmaceutical composition comprising at least one of such anti-CD160 antibody or fragment thereof, and a pharmaceutically acceptable carrier.

Additionally, the present invention relates to a method for inhibiting angiogenesis comprising providing to a patient in need thereof such a pharmaceutical composition.

Finally, the present invention relates to the use of at least one of such anti-CD160 antibody or fragment thereof for the preparation of a medicament for treating and/or preventing a pathology associated with endothelial cells proliferation engaged in an angiogenesis process.

### BRIEF DESCRIPTION OF THE FIGURES:

Figure 1: Amino acid sequence (SEQ ID No.6) and nucleotide sequence (SEQ ID No.7) of the variable region of the heavy chain of the mouse CD160 antibody.
Figure 2: Amino acid sequence (SEQ ID No.8) and nucleotide sequence (SEQ ID No.9) of the variable region of the light chain of the mouse CD160 antibody.
Figure 3: Amino acid sequence (SEQ ID No. 10) and nucleotide sequence (SEQ ID No.11) derived from the constant region of the heavy chain of human gamma 1 immunoglobulins by the insertion of an additional cysteine and of a stop codon.
Figure 4: Amino acid sequence (SEQ ID No. 12) and nucleotide sequence (SEQ ID No.13) of the constant region of the heavy chain of human gamma 1 immunoglobulins. The first AGC codon was modified in GCT in order to create a cloning site (NheI).
Figure 5: Amino acid sequence (SEQ ID No.14) and nucleotide sequence (SEQ ID No.15) of the constant region of the heavy chain of human gamma 4 immunoglobulins. The first AGC codon was modified in GCT in order to create a cloning site (NheI).
Figure 6: Amino acid sequence (SEQ ID No.16) and nucleotide sequence (SEQ ID No.17) of the constant region of the light chain of human kappa immunoglobulins.
Figure 7: Amino acid sequence (SEQ ID No.18) and nucleotide sequence (SEQ ID No.19) of a recombinant variable region of the light chain of the mouse CD160 antibody comprising the human VL signal peptide (shared).
Figure 8: Amino acid sequence (SEQ ID No.20) and nucleotide sequence (SEQ ID No.21) of a recombinant variable region of the heavy chain of the mouse CD160 antibody comprising the human VH signal peptide (shared).
Figure 9: Alignment between amino acid sequence the mouse variable light chain (SEQ ID No.8) and the human immunoglobulin having the maximum of homology (SEQ ID No. 22; Accession number AAZ09098). The CDR regions are identified (shared).
Figure 10: Alignment between amino acid sequence the mouse variable heavy chain (SEQ ID No.6) and one of the human immunoglobulin having the maximum of homology (SEQ ID No. 23; Accession number 3FCTB).The CDR regions are identified (shared).
Figure 11: Alignment between amino acid sequence the mouse variable heavy chain (SEQ ID No.6) and one of the human immunoglobulin having the maximum of homology (SEQ ID No. 24; Accession number AAG00910).The CDR regions are identified (shared).

### DETAILED DESCRIPTION:

In a first aspect, the present invention concerns an antibody, or fragment thereof, capable of binding to CD160, said antibody comprising:
a) a light chain comprising three light chain complementary regions (CDRs) having the following amino acid sequences:
   i) the light chain CDR1: QSISNH (SEQ ID NO:1);
   ii) the light chain CDR2: YAS;
   iii) the light chain CDR3: QQSNSWPLT (SEQ ID NO: 2); and
   a light chain framework sequence from an immunoglobulin light chain; and
b) a heavy chain comprising three heavy chain complementary regions (CDRs) having the following amino acid sequences:
   i) the heavy chain CDR1: GYTFTDYW (SEQ ID NO: 3);
   ii) the heavy chain CDR2: IYPGDDDA (SEQ ID NO: 4);
   iii) the heavy chain CDR3: ARRGIAAVVGGFDY (SEQ ID NO: 5); and
   a heavy chain framework sequence from an immunoglobulin heavy chain.

An antibody is an immunoglobulin molecule corresponding to a tetramer comprising four polypeptide chains, two identical heavy (H) chains (about 50-70 kDa when full length) and two identical light (L) chains (about 25 kDa when full length) inter-connected by disulfide bonds. Light chains are classified as kappa and lambda. Heavy chains are classified as gamma, mu, alpha, delta, or epsilon, and define the antibody's isotype as IgG, IgM, IgA, IgD, and IgE, respectively. Each heavy chain is comprised of a N-term heavy chain variable region (abbreviated herein as HCVR) and a heavy chain constant region. The heavy chain constant region is comprised of three domains (CH1, CH2, and CH3) for IgG, IgD, and IgA; and 4 domains (CH1, CH2, CH3, andCH4) for IgM and IgE. Each light chain is comprised of a N-term light chain variable region (abbreviated herein as LCVR) and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions (KABAT, "Sequences of Proteins of Immunological Interest", National Institutes of Health, Bethesda, Md., 1987 and1991; CHOTHIA et al., J. Mol. Biol., vol.196, p: 901-17, 1987; CHOTHIA et al., Nature, vol.342, p: 878-83, 1989). The functional ability of the antibody to bind a particular antigen depends on the variable regions of each light/heavy chain pair, and is largely determined by the CDRs.

The term "antibody", as used herein, refers to a monoclonal antibody *per se*. A monoclonal antibody can be a human antibody, chimeric antibody and/or humanized antibody.

The term "fragments" as used herein refers to antibody fragments that bind to a CD160. For example, antibody fragments capable of binding to CD160 include Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the invention.

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(ab')2 heavy chain portion can be designed to include DNA sequences encoding the CH₁ domain and/or hinge region of the heavy chain. The various portions of antibodies can be joined together chemically by conventional techniques, or can be prepared as a contiguous protein using genetic engineering techniques.

The expression "capable of binding to CD160" refers to a K_{D} of less than 10⁻⁷ M, preferably from less than 10⁻⁸ M, and more preferably less than 10⁻⁹ M for CD 160.

Preferably, but not necessarily, the antibodies useful in the invention are produced recombinantly, as manipulation of the typically murine or other non-human antibodies with the appropriate specificity is required in order to convert them to humanized form. Antibodies may or may not be glycosylated, though glycosylated antibodies are preferred. Antibodies are properly cross-linked via disulfide bonds, as is well-known.

As is well understood in the art, monoclonal antibodies can readily be generated with appropriate specificity by standard techniques of immunization of mammals forming produces it. These nucleotide sequences can then be manipulated to provide them in humanized form.

By "chimeric antibody" is meant an antibody that is composed of variables regions from a murine immunoglobulin and of constant regions of a human immunoglobulin. This alteration consists simply of substituting the constant region of a human antibody for the murine constant region, thus resulting in a human/murine chimera which may have sufficiently low immunogenicity to be acceptable for pharmaceutical use.

A number of methods for producing such chimeric antibodies have yet been reported, thus forming part of the general knowledge of the skilled artisan (See, e.g., U.S. Pat. No. 5,225,539).

In a preferred embodiment, said antibody is a chimeric antibody and the light and heavy chain framework sequences are from mouse immunoglobulin light and heavy chains respectively.

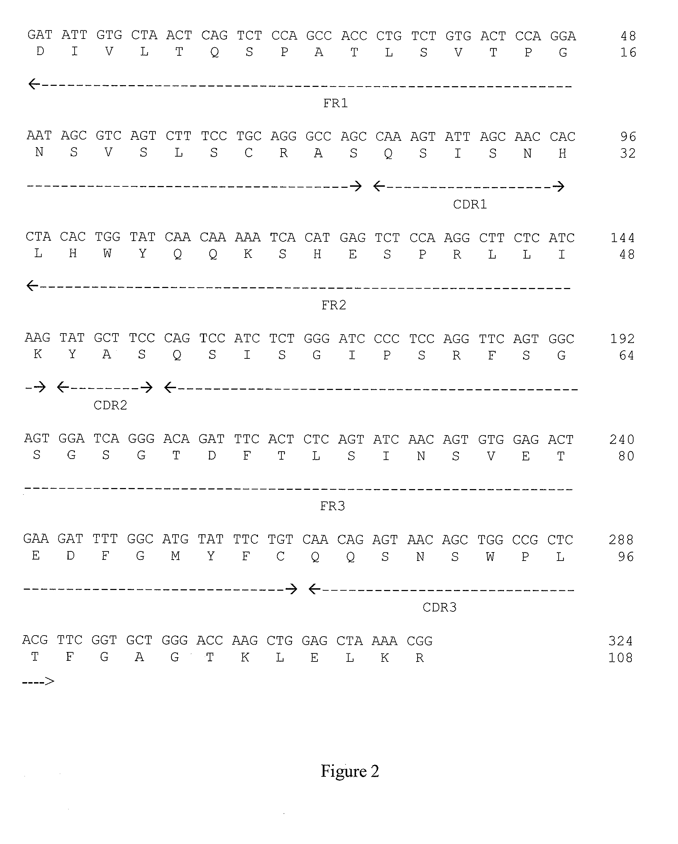

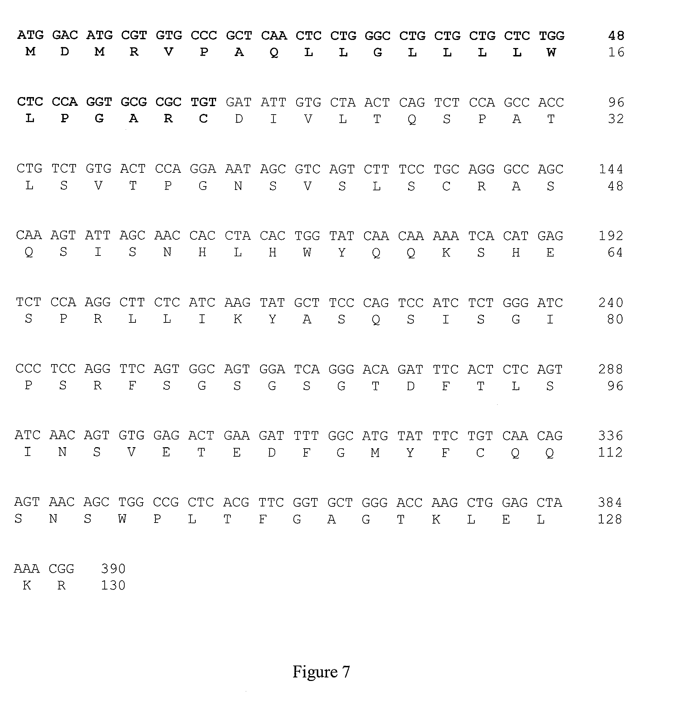

In a particular embodiment of this preferred embodiment, said antibody comprises the light chain variable region (LCVR) with the amino acid sequence SEQ ID NO: 8 shown in figure 2. Preferably, said antibody comprises the light chain variable region (LCVR) encoded by the nucleic acid sequence SEQ ID NO:9 shown in figure 2.
Advantageously, said antibody comprises the light chain variable region (LCVR) with the amino acid sequence SEQ ID NO: 18 shown in figure 7. Preferably, said antibody comprises the light chain variable region (LCVR) encoded by the nucleic acid sequence SEQ ID NO: 19 shown in figure 7.

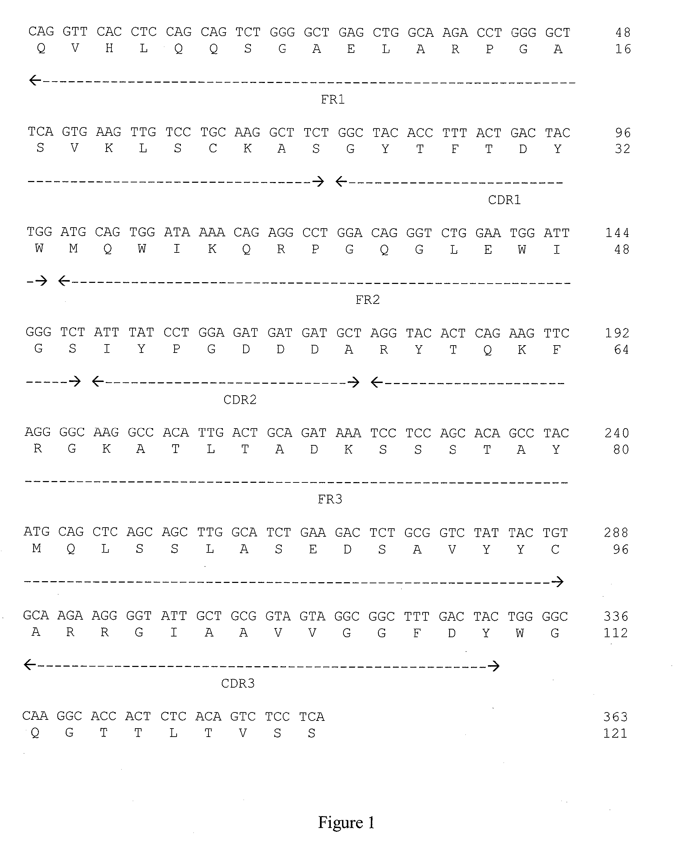

In another particular embodiment of this preferred embodiment, said antibody comprises the heavy chain variable region (HCVR) with the amino acid sequence SEQ ID NO:6 shown in figure 1. Preferably, said antibody comprises the heavy chain variable region (HCVR) encoded by the nucleic acid sequence SEQ ID NO:7 shown in figure 1.

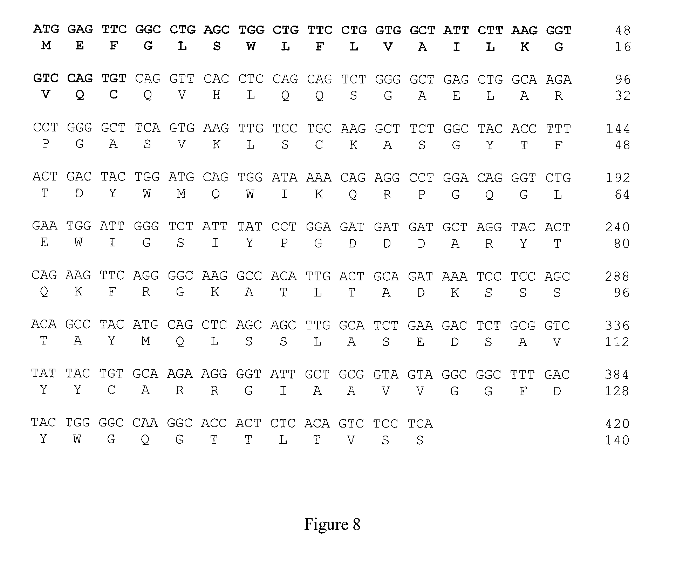

Advantageously, said antibody comprises the heavy chain variable region (HCVR) with the amino acid sequence SEQ ID NO: 20 shown in figure 8. Preferably, said antibody comprises the heavy chain variable region (HCVR) encoded by the nucleic acid sequence SEQ ID NO:21 shown in figure 8.

In still another particular embodiment of this preferred embodiment, said antibody further comprises the constant regions from human light and heavy chains.

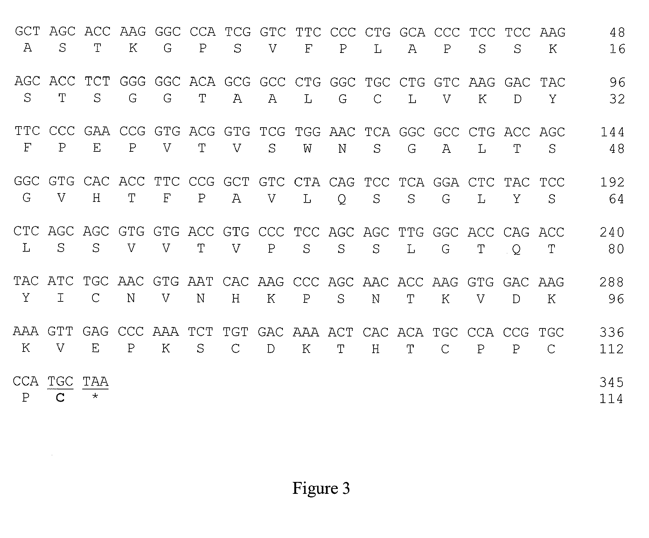

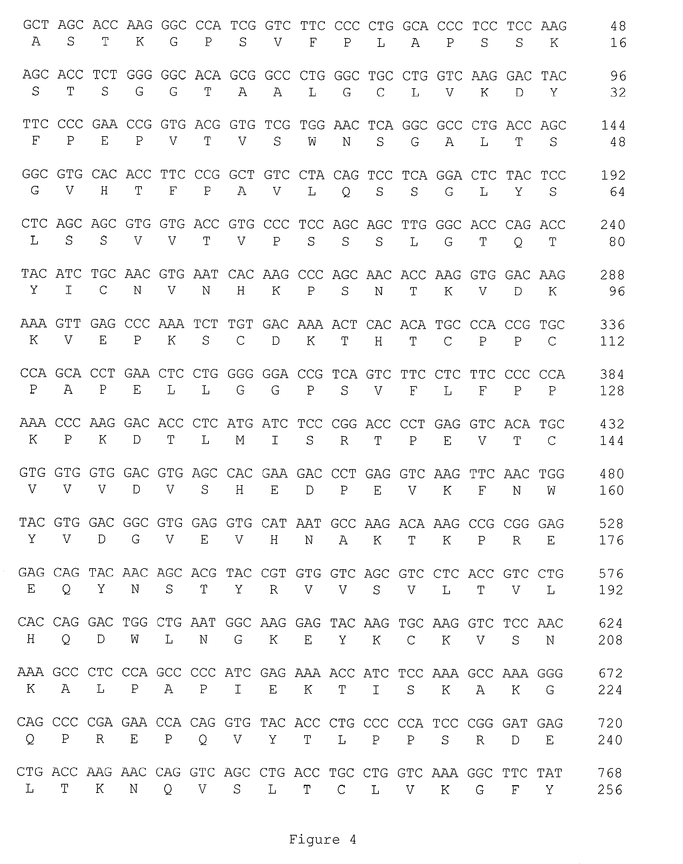

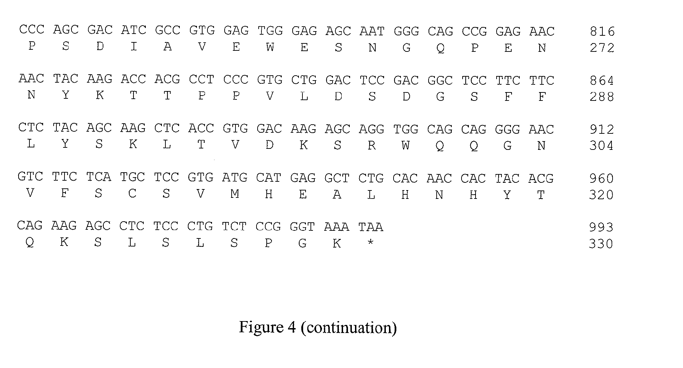

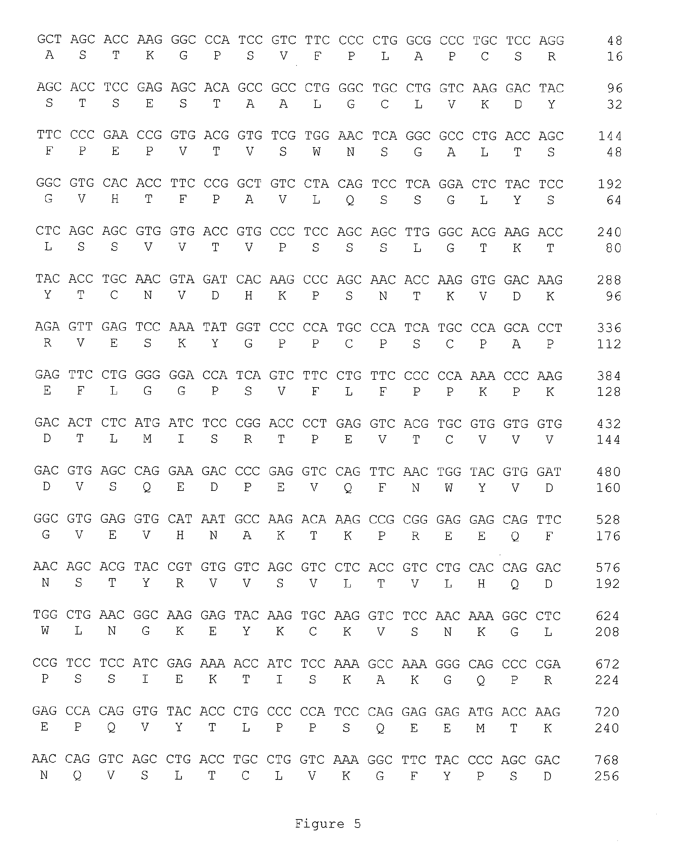

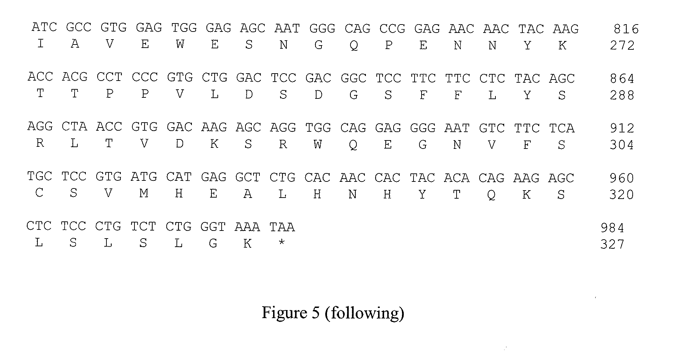

Advantageously, said antibody comprises a constant region from a human heavy chain selected in the group comprising:
- the recombinant constant region of heavy chain of human gamma 1 immunoglobulins shown in figure 3 comprising an insertion of an additional cysteine and of a stop codon (SEQ ID NO: 10);
- the recombinant constant region of heavy chain of human gamma 1 immunoglobulins shown in figure 4 (SEQ ID NO: 12); and
- the recombinant constant region of heavy chain of human gamma 4 immunoglobulins shown in figure 5 (SEQ ID NO: 14);

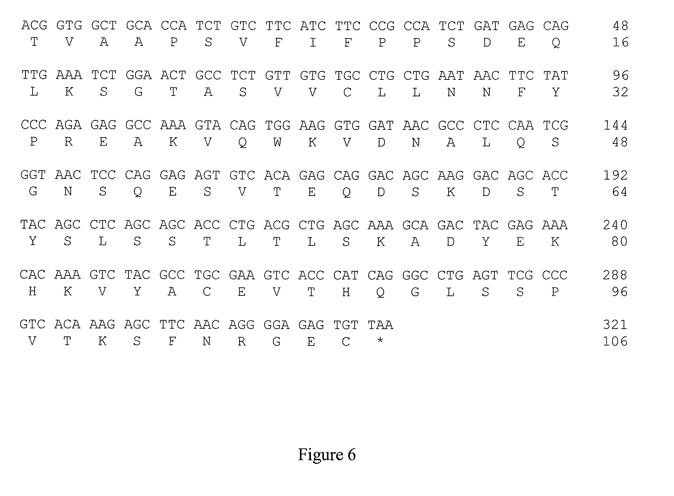

Again advantageously, said antibody comprises the constant region from a human light chain of human kappa immunoglobulins shown in figure 6 (SEQ ID NO: 16).

Nevertheless, murine antibodies and sometimes chimeric antibodies are recognized as foreign by a human host and elicit the so-called "human anti-mouse antibody" or "HAMA"response.

The term "humanized antibody" relates to an antibody wherein one or more amino acids of the mouse variable sequence have been substituted by the corresponding one or more amino acids of a homolog human variable sequence in order to reduce the immunogenicity of said mouse variable sequence after its administration to a human subject. This humanization of the variable region of the antibody and eventually the CDR is made by techniques that are by now well known in the art.

A number of methods for producing such humanized antibodies have yet been reported, thus forming part of the general knowledge of the skilled artisan.

As an example, British Patent Application GB 2188638A and US Patent No. 5,585,089 disclose processes wherein recombinant antibodies are produced where the only portion of the antibody that is substituted is the complementarity determining region, or "CDR". The CDR grafting technique has been used to generate antibodies which consist of murine CDRs, and human variable region framework and constant regions (See. e. g., RIECHMANN et al., Nature , vol.332, p: 323-327, 1988). These antibodies retain the human constant regions that are necessary for Fc dependent effector function, but are much less likely to evoke a HAMA response.

As another example, the framework regions of the variable regions are substituted by the corresponding human framework regions leaving the non-human CDR substantially intact, or even replacing the CDR with sequences derived from a human genome (See e.g. Patent application US 2006/258852). Fully human antibodies are produced in genetically modified mice whose immune systems have been altered to correspond to human immune systems. As mentioned above, it is sufficient for use in the methods of the invention, to employ an immunologically specific fragment of the antibody, including fragments representing single chain forms.

A humanized antibody again refers to an antibody comprising a human framework, at least one CDR from a non-human antibody, and in which any constant region present is substantially identical to a human immunoglobulin constant region, i. e., at least about 85 or 90%, preferably at least 95% identical. Hence, all parts of a humanized antibody, except possibly the CDRs, are substantially identical to corresponding parts of one or more native human immunoglobulin sequences. For example, a humanized immunoglobulin would typically not encompass a chimeric mouse variable region/human constant region antibody.

Humanized antibodies have at least three potential advantages over non-human and chimeric antibodies for use in human therapy :
1) Because the effector portion is human, it may interact better with the other parts of the human immune system (e.g., destroy the target cells more efficiently by complement-dependent cytotoxicity (CDC) or antibody-dependent cellular cytotoxicity (ADCC)).
2) The human immune system should not recognize the framework or C region of the humanized antibody as foreign, and therefore the antibody response against such an injected antibody should be less than against a totally foreign non-human antibody or a partially foreign chimeric antibody.
3) Injected non-human antibodies have been reported to have a half-life in the human circulation much shorter than the half-life of human antibodies. Injected humanized antibodies will have a half-life essentially identical to naturally occurring human antibodies, allowing smaller and less frequent doses to be given.

As an example, the design of humanized immunoglobulins may be carried out as follows: When an amino acid falls under the following category, the framework amino acid of a human immunoglobulin to be used (acceptor immunoglobulin) is replaced by a framework amino acid from a CDR-providing non-human immunoglobulin (donor immunoglobulin) : (a) the amino acid in the human framework region of the acceptor immunoglobulin is unusual for human immunoglobulin at that position, whereas the corresponding amino acid in the donor immunoglobulin is typical for human immunoglobulin at that position ; (b) the position of the amino acid is immediately adjacent to one of the CDRs ; or (c) any side chain atom of a framework amino acid is within about 5-6angstroms (center-to-center) of any atom of a CDR amino acid in a three dimensional immunoglobulin model (QUEEN et al., Proc. Natl. Acad. Sci. USA, vol.88, p:2869, 1991). When each of the amino acid in the human framework region of the acceptor immunoglobulin and a corresponding amino acid in the donor immunoglobulin is unusual for human immunoglobulin at that position, such an amino acid is replaced by an amino acid typical for human immunoglobulin at that position.

In another preferred embodiment, said antibody is a humanized antibody and the light and heavy chain framework sequences are from humanized immunoglobulin light and heavy chains respectively.

A preferred light chain variable region (LCVR) of a humanized antibody of the present invention has the amino acid sequence SEQ ID NO: 25, which amino acid sequence SEQ ID NO: 25 differs from at least one amino acid with SEQ ID NO:8.

This humanized sequence corresponds to the anti-CD 160 murine variable light chain in which one or more amino acid substitutions has been introduced in the framework after comparison with human immunoglobulin kappa variable light chain (SEQ ID NO: 22., Accession number AAZ09098) so as to reduce potential immunogenicity.

A preferred heavy chain variable region (HCVR) of a humanized antibody of the present invention has the amino acid sequence SEQ ID NO:26, which amino acid sequence SEQ ID NO:26 differs from at least one amino acid with SEQ ID NO:6.

This humanized sequence corresponds to the anti-CD160 murine variable heavy chain in which one or more amino acid substitutions has been introduced in the framework after comparison with human immunoglobulin variable heavy chains SEQ ID NO: 23 (Accession number 3FCTB) and SEQ ID NO: 24 (Accession number AAG00910) so as to reduce potential immunogenicity.

Preferably, said humanized antibody further comprises the constant regions from human light and heavy chains. As an example, said humanized antibody further comprises the constant regions from human light and heavy chains as described previously.

Other sequences are possible for the light and heavy chains for the humanized antibodies of the present invention. The immunoglobulins can have two pairs of light chain/heavy chain complexes, at least one chain comprising one or more mouse complementarity determining regions functionally joined to human framework region segments.

According to a second aspect, the present invention is related to a pharmaceutical composition comprising at least one anti-CD160 antibody or fragment thereof as described previously and a pharmaceutically acceptable carrier for use in diagnosis and/or prognosis and/or therapy.

Said composition is particularly useful for inhibiting angiogenesis, for the treatment of angiogenic pathologies, and preferably for inhibiting tumor progression.

Said composition may be in any pharmaceutical form suitable for administration to a patient, including but not limited to solutions, suspensions, lyophilized powders, capsule and tablets.

The pharmaceutical compositions of the invention may further comprise any pharmaceutically acceptable diluent, excipient or auxiliary.

The pharmaceutical composition of the invention may be formulated for injection, e.g. local injection, transmucosal administration, inhalation, oral administration and more generally any formulation that the skilled person finds appropriate to achieve the desired prognosis and/or diagnosis and/or therapy.

The anti-CD160 antibody of the invention is contained in said pharmaceutical composition in an amount effective to achieve the intended purpose, and in dosages suitable for the chosen route of administration.

More specifically, a therapeutically effective dose means an amount of a compound effective to prevent, alleviate or ameliorate symptoms of the disease or condition of the subject being treated, or to arrest said disease or condition.

Depending on the intended application, the anti-CD 160 antibody of the invention may further comprise additional constituents.

For example, when the anti-CD160 antibody of the invention is intended for prognosis or diagnosis, it may further comprise a detectable label, such as a fluorochrome, or an entity with enzymatic activity, or with radioactivity, and more generally any entity enabling the detection of said compound. The anti-CD 160 antibody of the invention may of course alternatively be used for the detection of anti-angiogenic sites.

The present invention hence also relates to a pharmaceutical composition or kit comprising at least one anti-CD 160 antibody of the invention, which is intended for the detection of anti-angiogenic sites.

When the anti-CD160 antibody of the invention is intended for therapeutic administration to an organism in need thereof, it may further comprise an immunotoxin and/or a radioelement.

A third aspect of the present invention concerns a method for inhibiting angiogenesis comprising providing to a patient in need thereof a pharmaceutical composition as described herein, which comprises at least one anti-CD160 antibody or fragment thereof as described previously.

As used herein, the term "patient" refers to a mammal, preferably to a human.

Preferably, a patient in need thereof corresponds to a patient suffering from a pathology associated with endothelial cells proliferation engaged in an angiogenesis process.

As an example of such pathologies, one can cites cancer (e.g. tumor vascularization), retinopathies (e.g., diabetic retinopathy), rheumatoid arthritis, chronic graft rejection such as cornea or kidney, acute graft rejection observed in xenograft, angioma, angiosarcoma such as Kaposi's sarcoma or Castelman syndrome, atherosclerosis, endometriosis associated with neovascularization, or tissue overproduction due to cicatrisation.

A forth aspect of the present invention concerns the use of at least one anti-CD 160 antibody or fragment thereof as described previously for the preparation of a medicament for treating and/or preventing a pathology associated with endothelial cells proliferation engaged in an angiogenesis process.

Such a pathology is selected in the group comprising cancer (e.g. tumor vascularization), retinopathies (e.g., diabetic retinopathy), rheumatoid arthritis, chronic graft rejection such as cornea or kidney, acute graft rejection observed in xenograft, angioma, angiosarcoma such as Kaposi's sarcoma or Castelman syndrome, atherosclerosis, endometriosis associated with neovascularization, or tissue overproduction due to cicatrisation.

Other embodiments and advantages of the present invention are illustrated in the following non-limiting examples.

### EXAMPLES

### 1) Production and purification of mAb:

MAbs are obtained by immunizing BALB/C mice with a fusion protein between GST and CD-160 (SEQ ID NO:27.; Accession number CAG46665). Cell fusions are carried out with NS1 as described in GOUTTEFANGEAS et al. (Eur. J. Immunol., vol.22, p:2681-5, 1992).

Screening is performed in two stages. Following indirect immunofluorescence staining and flow cytometry analysis, all hybridoma supernatants reacting with CD 160-GST fusion protein are retained. The cultures containing the selected mAb are cloned twice by limited dilutions.

### 2) Screening for an angiogenesis inhibition activity:

Growth reduced Matrigel (BD BIOSCIENCES) is diluted in collagen (1/6 v/v) and kept on ice. 160 µl of this solution is added to each well of 8-well culture slides precoated with type I rat tail collagen and left at 37°C for 1h. Following gel formation, a HUVEC suspension, mixed or not with control, FGF-2, sHLA-G1 or hybridoma supernatants is seeded on Matrigel/collagen gels for 24h at 37°C in a humidified 5% CO2 incubator.

Angiogenesis is quantified as described in RUGGERI et al. (Cancer Res., vol.63(18), p:5978-91, 2003).

Briefly, the culture medium is removed, the cells are rinsed twice with PBS and fixed for 30 min at room temperature in a 4% PFA solution. Then, the cells are washed twice with PBS and stained with Masson's Trichrom stain. The extent of microcapillary network is measured using an automated computer-assisted image analysis system (Imagenia, Biocom, Les Ulis, France), and the total length of the capillaries in each well is determined. The mean microcapillary network length (m) is calculated for each experimental condition. Experiments are performed in triplicate and repeated 3 times.

Suprisingly, one supernatant among all the hybridoma's supernatants leads to the inhibition of FGF-2 mediated tubule vessel growth demonstrating that the antibodies of this specific supernatant are able to inhibit angiogenesis.

### 3) Cloning of the variable regions of the anti-CD160 antibody having anti-angiogenesis properties:

The specific hybridoma producing antibodies inhibiting angiogenesis is isolated. Total RNA was extracted from cells of said hybridoma with RNeasy kit (QIAGEN) according to the instruction of the manufacturer.

50 µg of total RNA as described in CHARDES et al. (FEBS letters, vol.452, p:386-94, 1999) with primers specific of constant regions from the kappa light chain and from the gamma variable chain respectively. Then, the protocol described in CHARDES *et al*. (1999, abovementioned) was used for screening and isolating the variable regions of the immunoglobulin of interest.

This screening has enable to identify the variable regions of the light chain and of the heavy chain of the immunoglobulin of interest (figures 1 and 2).

These sequences were analysed by an alignment with other immunoglobulins on the IMGT/V-QUEST site (http://imgt.cines.fr/IMGT_vquest/share/textes/index.html) for identifying the framework region (FR) and the CDR position.

The CDR position as identified for the variable heavy and light chains are presented in figures 1 and 2 respectively.

### 4) humanization of the variable regions of the anti-CD160 antibody having anti-angiogenesis properties:

For the immunization of the identified variable regions are aligned with the human immunoglobulins having the maximum of homology with said variables regions.

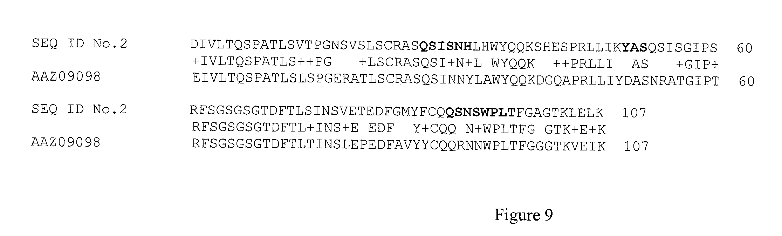

Human immunoglobulin having the maximum of homology (SEQ ID NO: 22; accession number AAZ09098) with the mouse variable light chain SEQ ID NO: 8. is shown in figure 9.

In view of this alignment, multiple substitutions are tested in order to humanize the mouse variable light chain. The tested substitutions are as follows:
* position 17 (of SEQ ID No.8): N -> E;
* position 18: S -> R
* position 19: V ->A
* position 41: H -> G
* position 53 : Q -> N
* position 80: T-> P
* position 84 : G ->A
* position 100: A -> G

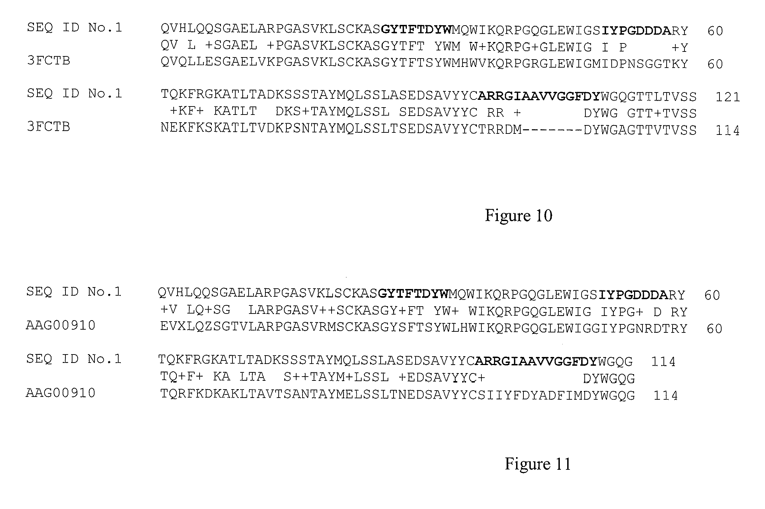

Human immunoglobulins having the maximum of homology (SEQ ID NO: 23 and SEQ ID NO: 24; accession number 3FCTB and AAG00910 respectively) with the mouse variable heavy chain SEQ ID No.1 are shown in figure 10 and 11 respectively.

In view of this alignment, multiple substitutions are tested in order to humanize the mouse variable heavy chain. The tested substitutions are as follows:
^{*} position 3 (of SEQ ID No.6): H -> Q;
^{*} position 35: Q -> H
^{*} position 87: A ->T

### 5) Construction of chimeric antibodies:

The sequences of the variable heavy and light chains, humanized or not, are combined with signal peptides and their respective human kappa and IgG1 or IgG4 constant region domains such as described in LEUNG et al. (Hybridoma, vol.13(6), p:469-76, 1994).

The anti-angiogenic activity of these antibodies is tested as described previously (cf. 2).

## Claims

1. An antibody or fragment thereof, capable of binding to CD160, said antibody comprising:
a) a light chain comprising three light chain complementary regions (CDRs) having the following amino acid sequences:
i) the light chain CDR1: QSISNH (SEQ ID NO:1);
ii) the light chain CDR2: YAS;
iii) the light chain CDR3: QQSNSWPLT (SEQ ID NO: 2); and
a light chain framework sequence from an immunoglobulin light chain; and
b) a heavy chain comprising three heavy chain complementary regions (CDRs) having the following amino acid sequences:
i) the heavy chain CDR1: GYTFTDYW (SEQ ID NO: 3);
ii) the heavy chain CDR2: IYPGDDDA (SEQ ID NO: 4);
iii) the heavy chain CDR3: ARRGIAAVVGGFDY (SEQ ID NO: 5); and
a heavy chain framework sequence from an immunoglobulin heavy chain.

2. The antibody of claim 1, wherein said antibody or fragment thereof is capable of binding to CD160 with a K_{D} of less than 10⁻⁷ M, preferably from less than 10⁻⁸ M.

3. The antibody of any one of claims 1 or 2, wherein said antibody comprises the light chain variable region (LCVR) with the amino acid sequence SEQ ID NO: 8.

4. The antibody of any one of claims 1 to 3, wherein said antibody comprises the heavy chain variable region (HCVR) with the amino acid sequence SEQ ID NO:6.

5. The antibody of any one of claims 1 or 2, wherein said antibody comprises a light chain variable region (LCVR) having the amino acid sequence SEQ ID NO: 25, which amino acid sequence SEQ ID NO: 25 differs from at least one amino acid with SEQ ID NO:8.

6. The antibody of any one of claims 1,2 or 5, wherein said antibody comprises a heavy chain variable region (HCVR) having the amino acid sequence SEQ ID NO:26, which amino acid sequence SEQ ID NO:26 differs from at least one amino acid with SEQ ID NO:6

7. The antibody of any one of claims 1 to 6, wherein said antibody further comprises the constant regions from human light and heavy chains.

8. The antibody of claim 7, wherein said antibody comprises:
a) a constant region from a human heavy chain selected in the group comprising:
• the recombinant constant region of heavy chain of human gamma 1 immunoglobulins having the sequence SEQ ID NO: 10, which sequence comprises an insertion of an additional cysteine and of a stop codon;
• the recombinant constant region of heavy chain of human gamma 1 immunoglobulins having the sequence SEQ ID NO: 12; and
• the recombinant constant region of heavy chain of human gamma 4 immunoglobulins having the sequence SEQ ID NO: 14; and
b) the constant region from human light chain of human kappa immunoglobulins having the sequence SEQ ID NO: 16.

9. The antibody of claim 8, wherein said antibody comprises the recombinant constant region of heavy chain of human gamma 1 immunoglobulins having the sequence SEQ ID NO: 10 and the constant region from human light chain of human kappa immunoglobulins having the sequence SEQ ID NO: 16.

10. A pharmaceutical composition comprising at least one antibody according to any one of claims 1 to 9, and a pharmaceutically acceptable carrier.

11. Use of at least one antibody as defined in any of claims 1 to 9, for the preparation of a medicament for treating and/or preventing a pathology associated with endothelial cells proliferation engaged in an angiogenesis process.

12. The use of claim 11, wherein said pathology is selected in the group comprising cancer, retinopathies, rheumatoid arthritis, chronic graft rejection, acute graft rejection observed in xenograft, angioma, angiosarcoma, atherosclerosis, endometriosis associated with neovascularization, and tissue overproduction due to cicatrisation.

13. The use of claim 12, wherein said pathology is selected in the retinopathies.

14. The use of claim 13, wherein the antibody is as defined in claim 9.


SEQUENCE LISTING

<110>  MONOCLONAL ANTIBODIES THERAPEUTICS

<120>  NEW ANTI-CD160 MONOCLONAL ANTIBODIES AND USES THEREOF

<130>  67883

<160>  27

<170>  PatentIn version 3.3

<210>  1
<211>  6
<212>  PRT
<213>  Artificial sequence

<220>
<223>  CDR1 from light chain of anti-CD160 antibody

<400>  1

Gln Ser Ile Ser Asn His
1               5


<210>  2
<211>  9
<212>  PRT
<213>  Artificial sequence

<220>
<223>  CDR3 from light chain of anti-CD160 antibody

<400>  2

Gln Gln Ser Asn Ser Trp Pro Leu Thr
1               5


<210>  3
<211>  8
<212>  PRT
<213>  Artificial sequence

<220>
<223>  CDR1 from heavy chain of anti-CD160 antibody

<400>  3

Gly Tyr Thr Phe Thr Asp Tyr Trp
1               5


<210>  4
<211>  8
<212>  PRT
<213>  Artificial sequence

<220>
<223>  CDR2 from heavy chain of anti-CD160 antibody

<400>  4

Ile Tyr Pro Gly Asp Asp Asp Ala
1               5


<210>  5

<211>  14
<212>  PRT
<213>  Artificial sequence

<220>
<223>  CDR3 from heavy chain of anti-CD160 antibody

<400>  5

Ala Arg Arg Gly Ile Ala Ala Val Val Gly Gly Phe Asp Tyr
1               5                   10


<210>  6
<211>  121
<212>  PRT
<213>  Mus musculus

<400>  6

Gln Val His Leu Gln Gln Ser Gly Ala Glu Leu Ala Arg Pro Gly Ala
1               5                   10                  15


Ser Val Lys Leu Ser Cys Lys Ala Ser Gly Tyr Thr Phe Thr Asp Tyr
            20                  25                  30


Trp Met Gln Trp Ile Lys Gln Arg Pro Gly Gln Gly Leu Glu Trp Ile
        35                  40                  45


Gly Ser Ile Tyr Pro Gly Asp Asp Asp Ala Arg Tyr Thr Gln Lys Phe
    50                  55                  60


Arg Gly Lys Ala Thr Leu Thr Ala Asp Lys Ser Ser Ser Thr Ala Tyr
65                  70                  75                  80


Met Gln Leu Ser Ser Leu Ala Ser Glu Asp Ser Ala Val Tyr Tyr Cys
                85                  90                  95


Ala Arg Arg Gly Ile Ala Ala Val Val Gly Gly Phe Asp Tyr Trp Gly
            100                 105                 110


Gln Gly Thr Thr Leu Thr Val Ser Ser
        115                 120


<210>  7
<211>  363
<212>  DNA
<213>  Mus musculus

<400>  7
caggttcacc tccagcagtc tggggctgag ctggcaagac ctggggcttc agtgaagttg      60

tcctgcaagg cttctggcta cacctttact gactactgga tgcagtggat aaaacagagg     120

cctggacagg gtctggaatg gattgggtct atttatcctg gagatgatga tgctaggtac     180

actcagaagt tcaggggcaa ggccacattg actgcagata aatcctccag cacagcctac     240

atgcagctca gcagcttggc atctgaagac tctgcggtct attactgtgc aagaaggggt     300

attgctgcgg tagtaggcgg ctttgactac tggggccaag gcaccactct cacagtctcc     360

tca                                                                 363

<210>  8
<211>  108
<212>  PRT
<213>  Mus musculus

<400>  8

Asp Ile Val Leu Thr Gln Ser Pro Ala Thr Leu Ser Val Thr Pro Gly
1               5                   10                  15

Asn Ser Val Ser Leu Ser Cys Arg Ala Ser Gln Ser Ile Ser Asn His
            20                  25                  30

Leu His Trp Tyr Gln Gln Lys Ser His Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Met Tyr Phe Cys Gln Gln Ser Asn Ser Trp Pro Leu
                85                  90                  95

Thr Phe Gly Ala Gly Thr Lys Leu Glu Leu Lys Arg
            100                 105

<210>  9
<211>  324
<212>  DNA
<213>  Mus musculus

<400>  9
gatattgtgc taactcagtc tccagccacc ctgtctgtga ctccaggaaa tagcgtcagt      60

ctttcctgca gggccagcca aagtattagc aaccacctac actggtatca acaaaaatca     120

catgagtctc caaggcttct catcaagtat gcttcccagt ccatctctgg gatcccctcc     180

aggttcagtg gcagtggatc agggacagat ttcactctca gtatcaacag tgtggagact     240

gaagattttg gcatgtattt ctgtcaacag agtaacagct ggccgctcac gttcggtgct     300

gggaccaagc tggagctaaa acgg                                            324

<210>  10
<211>  114
<212>  PRT
<213>  Homo sapiens

<400>  10

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Cys

<210>  11
<211>  345
<212>  DNA
<213>  Homo sapiens

<400>  11
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccat gctaa                     345

<210>  12
<211>  330
<212>  PRT
<213>  Homo sapiens

<400>  12

<210>  13
<211>  993
<212>  DNA
<213>  Homo sapiens

<400>  13
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa taa                                  993


<210>  14
<211>  327
<212>  PRT
<213>  Homo sapiens

<400>  14

Ala Ser Thr Lys Gly Pro Ser Val Phe Pro Leu Ala Pro Cys Ser Arg
1               5                   10                  15


Ser Thr Ser Glu Ser Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Lys Thr
65                  70                  75                  80


Tyr Thr Cys Asn Val Asp His Lys Pro Ser Asn Thr Lys Val Asp Lys

85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  15
<211>  984
<212>  DNA
<213>  Homo sapiens

<400>  15

gctagcacca agggcccatc cgtcttcccc ctggcgccct gctccaggag cacctccgag     60

agcacagccg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca acgtagatca caagcccagc aacaccaagg tggacaagag agttgagtcc     300

aaatatggtc ccccatgccc atcatgccca gcacctgagt tcctgggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat     480

ggcgtggagg tgcataatgc caagacaaag ccgcgggagg agcagttcaa cagcacgtac     540

cgtgtggtca gcgtcctcac cgtcctgcac caggactggc tgaacggcaa ggagtacaag     600

tgcaaggtct ccaacaaagg cctcccgtcc tccatcgaga aaaccatctc caaagccaaa     660

gggcagcccc gagagccaca ggtgtacacc ctgcccccat cccaggagga gatgaccaag     720

aaccaggtca gcctgacctg cctggtcaaa ggcttctacc ccagcgacat cgccgtggag     780

tgggagagca atgggcagcc ggagaacaac tacaagacca cgcctcccgt gctggactcc     840

gacggctcct tcttcctcta cagcaggcta accgtggaca agagcaggtg gcaggagggg     900

aatgtcttct catgctccgt gatgcatgag gctctgcaca accactacac acagaagagc     960

ctctccctgt ctctgggtaa ataa                                            984

<210>  16
<211>  106
<212>  PRT
<213>  Homo sapiens

<400>  16

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  17
<211>  321
<212>  DNA
<213>  Homo sapiens

<400>  17
acggtggctg caccatctgt cttcatcttc ccgccatctg atgagcagtt gaaatctgga      60

actgcctctg ttgtgtgcct gctgaataac ttctatccca gagaggccaa agtacagtgg     120

aaggtggata acgccctcca atcgggtaac tcccaggaga gtgtcacaga gcaggacagc     180

aaggacagca cctacagcct cagcagcacc ctgacgctga gcaaagcaga ctacgagaaa     240

cacaaagtct acgcctgcga agtcacccat cagggcctga gttcgcccgt cacaaagagc     300

ttcaacaggg gagagtgtta a                                               321


<210>  18
<211>  130
<212>  PRT
<213>  Mus musculus

<400>  18

Met Asp Met Arg Val Pro Ala Gln Leu Leu Gly Leu Leu Leu Leu Trp
1               5                   10                  15

Leu Pro Gly Ala Arg Cys Asp Ile Val Leu Thr Gln Ser Pro Ala Thr
            20                  25                  30

Leu Ser Val Thr Pro Gly Asn Ser Val Ser Leu Ser Cys Arg Ala Ser
        35                  40                  45

Gln Ser Ile Ser Asn His Leu His Trp Tyr Gln Gln Lys Ser His Glu
    50                  55                  60

Ser Pro Arg Leu Leu Ile Lys Tyr Ala Ser Gln Ser Ile Ser Gly Ile
65                  70                  75                  80

Pro Ser Arg Phe Ser Gly Ser Gly Ser Gly Thr Asp Phe Thr Leu Ser
                85                  90                  95

Ile Asn Ser Val Glu Thr Glu Asp Phe Gly Met Tyr Phe Cys Gln Gln
            100                 105                 110

Ser Asn Ser Trp Pro Leu Thr Phe Gly Ala Gly Thr Lys Leu Glu Leu
        115                 120                 125

Lys Arg
    130


<210>  19
<211>  390
<212>  DNA
<213>  Mus musculus

<400>  19

atggacatgc gtgtgcccgc tcaactcctg ggcctgctgc tgctctggct cccaggtgcg 60

cgctgtgata ttgtgctaac tcagtctcca gccaccctgt ctgtgactcc aggaaatagc 120

gtcagtcttt cctgcagggc cagccaaagt attagcaacc acctacactg gtatcaacaa 180

aaatcacatg agtctccaag gcttctcatc aagtatgctt cccagtccat ctctgggatc 240

ccctccaggt tcagtggcag tggatcaggg acagatttca ctctcagtat caacagtgtg 300

gagactgaag attttggcat gtatttctgt caacagagta acagctggcc gctcacgttc 360

ggtgctggga ccaagctgga gctaaaacgg 390

<210> 20
<211> 140
<212> PRT
<213> Mus musculus

<400> 20

Met Glu Phe Gly Leu Ser Trp Leu Phe Leu Val Ala Ile Leu Lys Gly
1               5                   10                  15

Val Gln Cys Gln Val His Leu Gln Gln Ser Gly Ala Glu Leu Ala Arg
            20                  25                  30

Pro Gly Ala Ser Val Lys Leu Ser Cys Lys Ala Ser Gly Tyr Thr Phe
        35                  40                  45

Thr Asp Tyr Trp Met Gln Trp Ile Lys Gln Arg Pro Gly Gln Gly Leu
    50                  55                  60

Glu Trp Ile Gly Ser Ile Tyr Pro Gly Asp Asp Asp Ala Arg Tyr Thr
65                  70                  75                  80

Gln Lys Phe Arg Gly Lys Ala Thr Leu Thr Ala Asp Lys Ser Ser Ser
                85                  90                  95

Thr Ala Tyr Met Gln Leu Ser Ser Leu Ala Ser Glu Asp Ser Ala Val
            100                 105                 110

Tyr Tyr Cys Ala Arg Arg Gly Ile Ala Ala Val Val Gly Gly Phe Asp
        115                 120                 125

Tyr Trp Gly Gln Gly Thr Thr Leu Thr Val Ser Ser
    130                 135                 140

<210> 21
<211> 420
<212> DNA
<213> Mus musculus

<400> 21
atggagttcg gcctgagctg gctgttcctg gtggctattc ttaagggtgt ccagtgtcag 60

gttcacctcc agcagtctgg ggctgagctg gcaagacctg gggcttcagt gaagttgtcc 120

tgcaaggctt ctggctacac ctttactgac tactggatgc agtggataaa acagaggcct 180

ggacagggtc tggaatggat tgggtctatt tatcctggag atgatgatgc taggtacact 240

cagaagttca ggggcaaggc cacattgact gcagataaat cctccagcac agcctacatg 300

cagctcagca gcttggcatc tgaagactct gcggtctatt actgtgcaag aaggggtatt 360

gctgcggtag taggcggctt tgactactgg ggccaaggca ccactctcac agtctcctca 420

<210> 22
<211> 107
<212> PRT
<213> Homo sapiens

<400> 22

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Ile Asn Asn Tyr
            20                  25                  30

Leu Ala Trp Tyr Gln Gln Lys Asp Gly Gln Ala Pro Arg Leu Leu Ile
        35                  40                  45

Tyr Asp Ala Ser Asn Arg Ala Thr Gly Ile Pro Thr Arg Phe Ser Gly
    50                  55                  60

Ser Gly Ser Gly Thr Asp Phe Thr Leu Thr Ile Asn Ser Leu Glu Pro
65                  70                  75                  80

Glu Asp Phe Ala Val Tyr Tyr Cys Gln Gln Arg Asn Asn Trp Pro Leu
                85                  90                  95

Thr Phe Gly Gly Gly Thr Lys Val Glu Ile Lys
            100                 105

<210> 23
<211> 114
<212> PRT
<213> Homo sapiens

<400> 23

Gln Val Gln Leu Leu Glu Ser Gly Ala Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Arg Gly Leu Glu Trp Ile
        35                  40                  45

Gly Met Ile Asp Pro Asn Ser Gly Gly Thr Lys Tyr Asn Glu Lys Phe
    50                  55                  60

Lys Ser Lys Ala Thr Leu Thr Val Asp Lys Pro Ser Asn Thr Ala Tyr

65                  70                  75                  80

Met Gln Leu Ser Ser Leu Thr Ser Glu Asp Ser Ala Val Tyr Tyr Cys
                85                  90                  95

Thr Arg Arg Asp Met Asp Tyr Trp Gly Ala Gly Thr Thr Val Thr Val
            100                 105                 110

Ser Ser

<210>  24
<211>  114
<212>  PRT
<213>  Homo sapiens

<220>
<221>  misc_feature
<222>  (3)..(3)
<223>  Xaa can be any naturally occurring amino acid

<400>  24

Glu Val Xaa Leu Gln Glx Ser Gly Thr Val Leu Ala Arg Pro Gly Ala
1               5                   10                  15

Ser Val Arg Met Ser Cys Lys Ala Ser Gly Tyr Ser Phe Thr Ser Tyr
            20                  25                  30

Trp Leu His Trp Ile Lys Gln Arg Pro Gly Gln Gly Leu Glu Trp Ile
        35                  40                  45

Gly Gly Ile Tyr Pro Gly Asn Arg Asp Thr Arg Tyr Thr Gln Arg Phe
    50                  55                  60

Lys Asp Lys Ala Lys Leu Thr Ala Val Thr Ser Ala Asn Thr Ala Tyr
65                  70                  75                  80

Met Glu Leu Ser Ser Leu Thr Asn Glu Asp Ser Ala Val Tyr Tyr Cys
                85                  90                  95

Ser Ile Ile Tyr Phe Asp Tyr Ala Asp Phe Ile Met Asp Tyr Trp Gly
            100                 105                 110

Gln Gly

<210>  25
<211>  108
<212>  PRT
<213>  Artificial sequence

<220>
<223>  humanized variable light chain anti-CD160 antibody

<220>
<221>  MISC_FEATURE
<222>  (17)..(17)
<223>  X= N or E

<220>
<221>  MISC_FEATURE
<222>  (18)..(18)
<223>  X= S or R

<220>
<221>  MISC_FEATURE
<222>  (19)..(19)
<223>  X= V or A

<220>
<221>  MISC_FEATURE
<222>  (41)..(41)
<223>  X= H or G

<220>
<221>  MISC_FEATURE
<222>  (53)..(53)
<223>  X= Q or N

<220>
<221>  MISC_FEATURE
<222>  (80)..(80)
<223>  X= T or P

<220>
<221>  MISC_FEATURE
<222>  (84)..(84)
<223>  X= G or A

<220>
<221>  MISC_FEATURE
<222>  (100)..(100)
<223>  X= G or A

<400>  25

Asp Ile Val Leu Thr Gln Ser Pro Ala Thr Leu Ser Val Thr Pro Gly
1               5                   10                  15

Xaa Xaa Xaa Ser Leu Ser Cys Arg Ala Ser Gln Ser Ile Ser Asn His
            20                  25                  30

Leu His Trp Tyr Gln Gln Lys Ser Xaa Glu Ser Pro Arg Leu Leu Ile
        35                  40                  45

Lys Tyr Ala Ser Xaa Ser Ile Ser Gly Ile Pro Ser Arg Phe Ser Gly
    50                  55                  60

Ser Gly Ser Gly Thr Asp Phe Thr Leu Ser Ile Asn Ser Val Glu Xaa
65                  70                  75                  80

Glu Asp Phe Xaa Met Tyr Phe Cys Gln Gln Ser Asn Ser Trp Pro Leu
                85                  90                  95

Thr Phe Gly Xaa Gly Thr Lys Leu Glu Leu Lys Arg
            100                 105

<210>  26
<211>  121
<212>  PRT
<213>  Artificial sequence

<220>
<223>  humanized variable heavy chain anti-CD160 antibody


<220>
<221>  MISC_FEATURE
<222>  (3)..(3)
<223>  X= H or Q

<220>
<221>  MISC_FEATURE
<222>  (35)..(35)
<223>  X= H or Q

<220>
<221>  MISC_FEATURE
<222>  (87)..(87)
<223>  X= A or T

<400>  26

Gln Val Xaa Leu Gln Gln Ser Gly Ala Glu Leu Ala Arg Pro Gly Ala
1               5                   10                  15


Ser Val Lys Leu Ser Cys Lys Ala Ser Gly Tyr Thr Phe Thr Asp Tyr
            20                  25                  30


Trp Met Xaa Trp Ile Lys Gln Arg Pro Gly Gln Gly Leu Glu Trp Ile
        35                  40                  45


Gly Ser Ile Tyr Pro Gly Asp Asp Asp Ala Arg Tyr Thr Gln Lys Phe
    50                  55                  60


Arg Gly Lys Ala Thr Leu Thr Ala Asp Lys Ser Ser Ser Thr Ala Tyr
65                  70                  75                  80


Met Gln Leu Ser Ser Leu Xaa Ser Glu Asp Ser Ala Val Tyr Tyr Cys
                85                  90                  95


Ala Arg Arg Gly Ile Ala Ala Val Val Gly Gly Phe Asp Tyr Trp Gly
            100                 105                 110


Gln Gly Thr Thr Leu Thr Val Ser Ser
        115                 120


<210>  27
<211>  181
<212>  PRT
<213>  Homo sapiens

<400>  27

Met Leu Leu Glu Pro Gly Arg Gly Cys Cys Ala Leu Ala Ile Leu Leu
1               5                   10                  15

Ala Ile Val Asp Ile Gln Ser Gly Gly Cys Ile Asn Ile Thr Ser Ser
            20                  25                  30

Ala Ser Gln Glu Gly Thr Arg Leu Asn Leu Ile Cys Thr Val Trp His
        35                  40                  45

Lys Lys Glu Glu Ala Glu Gly Phe Val Val Phe Leu Cys Lys Asp Arg
    50                  55                  60

Ser Gly Asp Cys Ser Pro Glu Thr Ser Leu Lys Gln Leu Arg Leu Lys
65                  70                  75                  80

Arg Asp Pro Gly Ile Asp Gly Val Gly Glu Ile Ser Ser Gln Leu Met
                85                  90                  95

Phe Thr Ile Ser Gln Val Thr Pro Leu His Ser Gly Thr Tyr Gln Cys
            100                 105                 110

Cys Ala Arg Ser Gln Lys Ser Gly Ile Arg Leu Gln Gly His Phe Phe
        115                 120                 125

Ser Ile Leu Phe Thr Glu Thr Gly Asn Tyr Thr Val Thr Gly Leu Lys
    130                 135                 140

Gln Arg Gln His Leu Glu Phe Ser His Asn Glu Gly Thr Leu Ser Ser
145                 150                 155                 160

Gly Phe Leu Gln Glu Lys Val Trp Val Met Leu Val Thr Ser Leu Val
                165                 170                 175

Ala Leu Gln Ala Leu
            180